# EUROPEAN PATENT APPLICATION

(11) **EP 4 527 376 A1**
(43) Date of publication of application: **26.03.2025**
(21) Application number: 23806766.4
(22) Date of filing: 09.05.2023
(51) Int. Cl.: A61K 9/16, A61K 9/52, A61K 31/428, A61K 47/34, A61K 47/32, A61P 25/16

(54) **LONG-ACTING SUSTAINED-RELEASE PREPARATION CONTAINING PRAMIPEXOLE AND PREPARATION METHOD THEREFOR**

(30) Priority: 16.05.2022 CN 202210528133
(71) Applicant: Sichuan Kelun Pharmaceutical Research Institute Co., Ltd., Chengdu, Sichuan 611138 (CN)
(72) Inventor: SU, Zhengxing, Chengdu, Sichuan 611138 (CN); DING, Duohao, Chengdu, Sichuan 611138 (CN); CHEN, Zhihao, Chengdu, Sichuan 611138 (CN); ZHAO, Dong, Chengdu, Sichuan 611138 (CN)
(74) Representative: Hoffmann Eitle
(86) International application number: PCT/CN2023/092960
(87) International publication number: WO 2023/221814

(57) **Abstract**

Disclosed is a long-acting sustained-release preparation pharmaceutical composition containing pramipexole, comprising a pharmaceutically acceptable salt of pramipexole and a pharmaceutical high polymer material. The preparation is selected from a microsphere, a sustained-release particle, and a subcutaneous implant. The pharmaceutically acceptable salt of pramipexole is selected from erucate, stearate, oleate, α-linolenate, heptadecanoate, pamoate, and palmitate. The high polymer material is selected from PLGA, PLA, mPEG-PLA, and PEG-PLA-PEG. The present invention belongs to the technical field of pharmaceutical preparations and solves the problems of short release period and poor release effect of pramipexole in the prior art.

## Description

### FIELD OF THE INVENTION

The present invention relates to the field of pharmaceutical formulation technology, and more specifically, it relates to a long-acting sustained-release formulation containing pramipexole and preparation method therefor.

### BACKGROUND OF THE INVENTION

According to the study by Qi S, Yin P, Wang L, et al. Prevalence of Parkinson's Disease: A Community-Based Study in China. Mov Disord. Published online August 14, 2021:mds.28762. doi:10.1002/mds.28762, over the past few decades, China has undergone significant socio-economic and demographic changes, entering a stage of deep aging. The prevalence rate of Parkinson's Disease (PD) in the population over 60 years old is 1.37% (95% confidence interval 1.02%-1.73%), and it is estimated that the total number of people with PD in China could be as high as 3.62 million.

Pramipexole is a second-generation potent, selective non-ergot dopamine D2 receptor agonist, used to treat primary Parkinson's disease in clinic, and can cover the entire stage of the disease treatment until the late stage. It can significantly improve the motor symptoms of early and late-stage Parkinson's disease patients and also improve the depressive symptoms in the Parkinson's disease patients. Pramipexole is the drug of first choice recommended by domestic and international Parkinson's disease treatment guidelines.

Patients with Parkinson's disease often suffer from symptoms such as memory decline, tremors in hands and feet, and difficulty swallowing, which leads to a common occurrence of missed doses and results in worsening conditions. The current pramipexole hydrochloride tablets on the market, due to their short release period, require administration three times a day, which is frequent and causes great inconvenience to patients with Parkinson's disease. Therefore, researching and developing pramipexole sustained-release formulations that can release the drug stably over a long period, such as administering once a week, once every two weeks, once a month, or even once every three months, is of great significance for improving patient compliance and enhancing clinical therapeutic efficacy.

### SUMMARY OF THE INVENTION

In light of the above problems, the present invention aims to provide a long-acting sustained-release formulation containing pramipexole with stable drug loading, high encapsulation efficiency, capable of achieving steady drug release over an extended time, and keeping the burst release to a very low limit, which is beneficial for clinical efficacy and medication safety.

In one aspect, the present invention provides a long-acting sustained-release formulation containing pramipexole, which comprises a pharmaceutically acceptable salt of pramipexole and a pharmaceutical high polymer material;
the formulation is selected from a microsphere, a sustained-release particle, and a subcutaneous implant;
the pharmaceutically acceptable salt of pramipexole is selected from erucate, stearate, oleate, α-linolenate, heptadecanoate, pamoate, and palmitate; preferably, the pharmaceutically acceptable salt of pramipexole is selected from erucate, stearate, oleate, α-linolenate, heptadecanoate, and palmitate; more preferably, the pharmaceutically acceptable salt of pramipexole is selected from erucate, stearate, oleate, heptadecanoate, and palmitate;
the high polymer material is selected from PLGA, PLA, mPEG-PLA, and PEG-PLA-PEG.

In certain embodiments, in the pharmaceutically acceptable salt of pramipexole provided by the present invention, the molar ratio of pramipexole to erucic acid, stearic acid, oleic acid, α-linolenic acid, heptadecanoic acid, or palmitic acid is 1:1.

In certain embodiments, in the pharmaceutically acceptable salt of pramipexole provided by the present invention, the molar ratio of pramipexole to pamoic acid is 1:1 or 2:1; preferably, the molar ratio of pramipexole to pamoic acid is 2:1.

In certain embodiments, in the sustained-release formulation provided by the present invention, the content of pramipexole (calculated as the free compound) constitutes 10-55% of the total weight of the formulation; and the content of the high polymer material constitutes 90-45% of the total weight of the formulation.

In certain embodiments, in the sustained-release formulation provided by the present invention, the high polymer material is PLGA, with a molecular weight of 10000-100000 Da, wherein the LA:GA block ratio of PLGA is 5:95 to 95:5.

In certain preferred embodiments, in the sustained-release formulation provided by the present invention, the high polymer material is PLGA, with a molecular weight of 25000-100000 Da, wherein the LA:GA block ratio of PLGA is 50:50 to 85:15.

In certain preferred embodiments, in the sustained-release formulation provided by the present invention, the type of the PLGA employed is 2.5A, 3A, 5A, 4.5A, 4.5E, 5E, 7A, 7E, or 8E.

In certain embodiments, in the sustained-release formulation provided by the present invention, the high polymer material is PLA, with a molecular weight of 10000-100000 Da.

In certain embodiments, in the sustained-release formulation provided by the present invention, the high polymer material is mPEG-PLA, wherein the molecular weight of mPEG in mPEG-PLA is 1000-4000 Da, and the molecular weight of PLA is 10000-100000 Da.

In certain embodiments, in the sustained-release formulation provided by the present invention, the high polymer material is PEG-PLA-PEG, wherein the molecular weight of PEG is 1000-4000 Da, and the molecular weight of PLA is 10000-100000 Da.

In a second aspect, the present invention provides a long-acting sustained-release formulation containing pramipexole, wherein the formulation is in the form of microspheres, and the active ingredient is pramipexole heptadecanoate, palmitate, erucate, stearate, or oleate.

In a second aspect, the present invention provides a long-acting sustained-release formulation containing pramipexole, wherein the formulation is in the form of microspheres, with the active ingredient being pramipexole heptadecanoate or palmitate; each unit formulation comprises 1~3 parts of pramipexole calculated as the free compound and 9~7 parts of PLGA, with the LA:GA block ratio of PLGA being 50:50, and the theoretical drug loading is 10%~30%, preferably, the theoretical drug loading is 15%~25%.

In certain embodiments, in the microspheres containing pramipexole provided by the present invention, the PLGA is 50:50 3A or 50:50 4.5E.

In a third aspect, the present invention provides a long-acting sustained-release formulation containing pramipexole, wherein the formulation is in the form of microspheres, with the active ingredient being pramipexole erucate, stearate or oleate; each unit formulation comprises 2~4 parts of pramipexole calculated as the free compound and 8~6 parts of PLGA, with the LA:GA block ratio of PLGA being 75:25-85:15, and the theoretical drug loading is 20%~40%, preferably, the theoretical drug loading is 25%~35%.

In certain embodiments, in the microspheres containing pramipexole provided by the present invention, the PLGA is 2.5A, 5A, 5E, or 7E; preferably, the PLGA is 2.5A, 5A, or 7E.

In certain embodiments, in the microspheres containing pramipexole provided by the present invention, the PLGA is 85:15 2.5A, 75:25 5E, 75:25 5A, or 75:25 7E; preferably, the PLGA is 85:15 2.5A, 75:25 5A, or 75:25 7E.

In the fourth aspect, the present invention provides a long-acting sustained-release formulation containing pramipexole, wherein the formulation is in the form of microspheres, with the active ingredient being pramipexole erucate, palmitate or oleate; each unit formulation comprises 3~5.5 parts of pramipexole calculated as the free compound and 7~4.5 parts of PLGA, with the LA:GA block ratio of PLGA being 75:25-85:15, and the theoretical drug loading is 30%~55%, preferably, the theoretical drug loading is 35%~50%, and more preferably, the theoretical drug loading is 35%~45%.

In certain embodiments, in the microspheres containing pramipexole provided by the present invention, the PLGA is 4.5A, 7A, 5E, or 8E; preferably, the PLGA is 4.5A, 7A, or 8E.

In certain embodiments, in the microspheres containing pramipexole provided by the present invention, the PLGA is 85:15 4.5A, 85:15 5E, 75:25 7A, or 75:25 8E; preferably, the PLGA is 85:15 4.5A, 75:25 7A, or 75:25 8E.

In the fifth aspect, the present invention provides a long-acting sustained-release formulation containing pramipexole, wherein the formulation is in the form of sustained-release particles, with the active ingredient being pramipexole erucate, pamoate, stearate, oleate, palmitate, or heptadecanoate; each unit formulation comprises 1~5.5 parts of pramipexole calculated as the free compound and 9~4.5 parts of PLGA, with the LA:GA block ratio of PLGA being 50:50-85:15, and the theoretical drug loading is 20%~55%.

In certain embodiments, in the sustained-release particles containing pramipexole provided by the present invention, the PLGA is 2.5A, 3A, 5A, 4.5A, 4.5E, 5E, 7A, or 7E.

In the sixth aspect, the present invention provides a long-acting sustained-release formulation containing pramipexole, wherein the formulation is in the form of a subcutaneous implant, with the active ingredient being pramipexole erucate, pamoate, stearate, oleate, or palmitate; each unit formulation comprises 2~5.5 parts of pramipexole calculated as the free compound and 8~4.5 parts of PLGA, with the LA:GA block ratio of PLGA being 50:50-85:15 (preferably 75:25-85:15), and the theoretical drug loading is 20%~55%.

In certain embodiments, in the subcutaneous implant containing pramipexole provided by the present invention, the PLGA is 2.5A, 4.5A, 5A, 5E, 7A, or 7E.

In the seventh aspect, the present invention provides a method for preparing the aforementioned microsphere formulation, comprising the following steps:
oil phase preparation: dissolving a pharmaceutically acceptable salt of pramipexole in a first solvent to obtain a first oil phase; dissolving PLGA in a second solvent to obtain a second oil phase;
external aqueous phase preparation: dissolving PVA in water for later use;
oil phase mixing: stirring and mixing the first oil phase and the second oil phase to obtain a mixed oil phase;
emulsification: filling the external aqueous phase into an inline shear machine and mixing it with the mixed oil phase at a speed of 5000 to 13000 rpm for shear emulsification;
solidification: evaporating the solvent from the sheared solution under stirring and heating according to a temperature increase curve to solidify and then lyophilizing to form the microspheres.

In preferred embodiments, during the solidification step, evaporating the solvent from the sheared solution under stirring and heating according to a temperature increase curve to solidify, sieving, and then lyophilizing to form the microspheres.

In certain embodiments, in the method for preparing microspheres provided by the present invention, the first solvent is selected from one or more of dichloromethane, benzyl alcohol, methanol, ethanol, isopropanol, *tert*-butanol, NMP, DMSO, and DMF; preferably dichloromethane or benzyl alcohol.

In certain embodiments, in the method for preparing microspheres provided by the present invention, the second solvent is selected from one or more of dichloromethane, chloroform, and ethyl acetate; preferably dichloromethane or chloroform.

In certain embodiments, in the method for preparing microspheres provided by the present invention, the mass concentration of pramipexole in the first oil phase (g/ml) is 0.05 to 0.9, preferably 0.05 to 0.7; the mass concentration of PLGA in the second oil phase (g/ml) is 0.1 to 0.4.

In the eighth aspect, the present invention provides a method for preparing the aforementioned sustained-release particles, comprising the following steps:
mixing a pharmaceutically acceptable salt of pramipexole with PLGA and adding the mixture to the feed hopper of a hot melt extruder;
extrusion: using a hot melt extruder to extrude at an extrusion speed of 100 rpm under a pressure of 60 bar, controlling the torque at 7~8 N.cm;
granulating: traction drawing after extrusion, and granulating with a pelletizer;
milling: crushing the granulated product with a low-temperature ball mill to a size of 50~100µm, preferably 60~100um, to obtain sustained-release particles.

In certain embodiments, in the method for preparing sustained-release particles, during the extrusion step, both the feed temperature and the pushing temperature are at room temperature, the mixing temperature is controlled at 70-140°C, the degassing temperature is the same as the mixing temperature, the extrusion temperature is controlled at 55-120°C, and the die orifice temperature is controlled at 50-75°C.

In certain embodiments, in the method for preparing sustained-release particles, during the extrusion step, both the feed temperature and the pushing temperature are at room temperature, the mixing temperature is controlled at 100-110°C, the degassing temperature is the same as the mixing temperature, the extrusion temperature is controlled at 85-110°C, and the die orifice temperature is controlled at 65-72°C.

In the ninth aspect, the present invention provides a method for preparing the aforementioned subcutaneous implant, comprising the following steps:
mixing a pharmaceutically acceptable salt of pramipexole with PLGA and adding the mixture to the feed hopper of a hot melt extruder;
extrusion: using a hot melt extruder to extrude at an extrusion speed of 100 rpm under a pressure of 60 bar, controlling the torque at 7~8 N.cm;
granulating: traction drawing after extrusion, and granulating with a pelletizer to obtain the subcutaneous implant.

In certain embodiments, in the method for preparing the subcutaneous implant, during the extrusion step, both the feed temperature and the pushing temperature are at room temperature, the mixing temperature is controlled at 70-140°C, the degassing temperature is the same as the mixing temperature, the extrusion temperature is controlled at 55-120°C, and the die orifice temperature is controlled at 65-75°C.

In certain embodiments, in the method for preparing the subcutaneous implant, during the granulation step, the die orifice size is set as 3mm, and the cutting length is set as 35mm.

### DETAILED DESCRIPTION OF THE INVENTION

### Definitions and Terminology

As used in the present invention, "microsphere" refers to a tiny spherical formulation formed by dispersing a drug within a high polymer matrix.

As used in the present invention, "sustained-release particle", also known as long-acting sustained-release particle or long-acting sustained-release microparticle, is a type of formulation that, in contrast to microspheres, also involves dispersing a drug within a high polymer matrix but obtained through methods such as milling into tiny particles. Compared to microspheres, sustained-release particles are irregular polyhedral in shape.

As used in the present invention, "subcutaneous implant," also known as subcutaneous insertion agent, refers to a type of drug delivery formulation shaped into rods that are implanted under the skin to slowly release the drug.

Regarding the high polymer materials involved in the present invention, the Chinese name for PLGA is polylactic acid-glycolic acid copolymer, wherein LA stands for lactic acid, and GA stands for glycolic acid; the Chinese name for PLA is poly(lactic acid); the Chinese name for mPEG-PLA is methoxy polyethylene glycol-poly(lactic acid); the Chinese name for PEG-PLA-PEG is poly(ethylene glycol)-poly(lactic acid)-poly(ethylene glycol); the Chinese name for PEG is poly(ethylene glycol).

Concerning the high polymer materials involved in the present invention, 5050 PLGA 3A is abbreviated as 5050 3A, wherein "5050" indicates the LA:GA block ratio of 50:50, "3" indicates the product intrinsic viscosity (IV) of about 0.3 dL/g, "A" denotes carboxyl end-capping, "E" indicates ester end-capping. PLA 2A indicates PLA with an IV of about 0.2 dL/g and carboxyl end-capping. mPEG-PLA 2000-20000 represents a two-block copolymer of PLA and mPEG, wherein the mPEG has a molecular weight of 2000 Da and PLA has a molecular weight of 20000 Da.

For the high polymer materials involved in the present invention, the molecular weight of the polymer is its statistical average value. Although the molecular weights stated in the invention are specific values, they actually represent a range for the high polymer materials, typically this range value is the stated molecular weight value ± 10%. For example, a molecular weight of 50000 Da actually has a range of 45000 Da to 55000 Da; a molecular weight of 100000 Da actually has a range of 90000 Da to 110000 Da, and so on.

As described in the present invention, the theoretical drug loading refers to the ratio of the API to the high polymer material (e.g., PLGA) in the feed, with the theoretical drug loading = weight of API / (weight of API + weight of high polymer material) * 100%, wherein the API is calculated as the free compound of pramipexole.

As used in the present invention, encapsulation efficiency = actual drug loading / theoretical drug loading * 100%.

As used herein, the "parts" mentioned when describing the content of the formulation components refer to weight parts.

As used herein, the molecular weight of the high polymer material refers to the weight average molecular weight.

As used in the present invention, the Chinese name for PVA is polyvinyl alcohol.

### BRIEF DESCRIPTION OF THE DRAWINGS

Figure 1: *in vivo* pharmacokinetic curves (Formulations 1, 2, 11)
Figure 2: enlarged graph of 24-hour release curves (Formulations 1, 2, 11)
Figure 3: *in vivo* pharmacokinetic release curves (Formulations 27, 38)
Figure 4: enlarged graph of 24-hour *in vivo* release curves (Formulations 27, 38)
Figure 5: *in vivo* pharmacokinetic release curves (Formulations 53, 61)
Figure 6: enlarged graph of 24-hour *in vivo* release curves (Formulations 53, 61).

### MODE OF CARRYING OUT THE INVENTION

To make the technical solutions and advantages of the present invention clearer, the technical solutions in the examples of the present invention will be described clearly and completely below. Unless specific conditions are stated in the examples, they are carried out under normal conditions or according to the manufacturer's recommended conditions. Reagents or instruments not specified with manufacturers are all conventional products available commercially.

The Chinese names for the English chemical abbreviations involved in the examples of the present invention are as follows:

| English abbreviation | Chinese name | English abbreviation | Chinese name |
|---|---|---|---|
| NMP | N-methylpyrrolidone | PDI | polydispersity index |
| DMF | N,N-dimethylformamide | Mw | Weight average molecular weight |
| DMSO | dimethyl sulfoxide | Span | Radial distance (span) |

The molecular weight of the high polymer formulations is primarily characterized by Mw and PDI. PDI indicates the distribution of molecular weight, generally between 1.5 and 2.0, indicating a uniform distribution of molecular weight.

### Example 1: Preparation of Microsphere Formulation Containing Pramipexole

**Table 1: Microsphere Formulation Feed Table**

| Formulation No. | | 1 | 2 | 19 | 21 | 22 | 43 | 45 | 46 |
|---|---|---|---|---|---|---|---|---|---|
| Salt Type | | heptadec anoate | palmitate | erucate | stearate | oleate | erucate | palmita te | oleate |
| Oil Phase 1 | API (g) | 1.5 | 4 | 6 | 4.7 | 3 | 13 | 10.5 | 5 |
| | Type of Solvent 1 | dichloro methane | benzyl alcohol | NMP | DMF | methano l | dichlor ometha ne | DMSO | benzyl alcoho 1 |
| | Solvent 1 (ml) | 30 | 30 | 15 | 15 | 15 | 15 | 15 | 15 |
| Oil Phase 2 | PLGA Type | 5050 3A | 5050 4.5E | 8515 2.5A | 7525 5A | 7525 7E | 8515 4.5A | 7525 7A | 7525 8E |
| | PLGA (g) | 12 | 12 | 11 | 12 | 12 | 12 | 12 | 11 |
| | Type of Solvent 2 | dichloro methane | dichlorom ethane | dichloro methane | dichloro methane | dichloro methane | dichlor ometha ne | dichlor ometha ne | dichlo romet hane |
| | Solvent 2 (ml) | 35 | 35 | 35 | 35 | 35 | 35 | 35 | 35 |
| Extern al Aqueo us Phase | PVA (g) | 60 | 60 | 30 | 30 | 30 | 30 | 30 | 30 |
| | Water (L) | 6 | 6 | 3 | 3 | 3 | 3 | 3 | 3 |
| Theoretical Drug Loading | | 11.1% | 25.0% | 35.3% | 28.1% | 20.0% | 52.0% | 46.7% | 31.3% |

### Preparation Method:

Oil phase preparation: The API starting material was weighed according to the above formulation feed table, with the feed amount of API calculated based on the free compound of pramipexole. The molar ratio of pramipexole to the various anions of the salts in Table 1 was 1:1. Solvent 1 was added, and the first oil phase was obtained upon dissolution. The prescribed amount of PLGA was weighed and added to solvent 2, which was then dissolved to obtain the second oil phase.

External aqueous phase preparation: The prescribed amount of PVA was weighed and added to water. It was dissolved for later use.

Oil phase mixing: The first oil phase and the second oil phase were stirred and mixed at a temperature between 4-40°C for 5-50 minutes to obtain a mixed oil phase.

Emulsification: The external aqueous phase was first filled into an inline shear machine (IKA 2000/03+DR shear head). The external aqueous phase and mixed oil phase were mixed at a speed of 8000 rpm for shear emulsification.

Solidification: the solvent was evaporated from the sheared solution under stirring and heating according to a temperature increase curve to solidify. It was sieved, and then lyophilized.

### Example 2: Preparation of Sustained-Release Particle Formulation Containing Pramipexole

**Table 2: Sustained-Release Particle Formulation Feed Table**

| Formulation No. | | 10 | 11 | 27 | 28 | 29 | 30 | 51 | 52 | 53 | 54 |
|---|---|---|---|---|---|---|---|---|---|---|---|
| Salt Type | | heptad ecanoa te | palm itate | erucat e | pamoat e | stearat e | oleate | eruc ate | pamo ate | palmit ate | oleat e |
| Startin g Materi al | API (g) | 3.8 | 4 | 5.5 | 3.6 | 4.7 | 4.7 | 13 | 7.5 | 10.5 | 10 |
| | Melting Point | 101.7 | 95.5 | 58 | 142 | 82 | 32 | 58 | 142 | 82 | 32 |
| Excipi ent | PLGA Type | 5050 3A | 5050 4.5E | 8515 4.5A | 7525 5E | 7525 5A | 7525 5E | 8515 2.5A | 7525 7E | 7525 7A | 752 5 7E |
| | Glass Transitio n Temperat ure | 32 | 45 | 47 | 49 | 47 | 51 | 47 | 49 | 47 | 51 |
| | PLGA (g) | 12 | 12 | 12 | 12 | 12 | 12 | 12 | 12 | 12 | 12 |
| Theoretical Drug Loading | | 24.0% | 25% | 31.4% | 23.1% | 28.1% | 28.1 % | 52% | 38.5 % | 46.7% | 45.5 % |
| Tempe rature Zone Setting (°C) | feed temperatu re | room temper ature | room temp eratu re | room tempe rature | room temper ature | room temper ature | room tempe rature | roo m temp eratu re | room temp eratu re | room temper ature | roo m tem pera ture |
| | pushing temperatu re | room temper ature | room temp eratu re | room tempe rature | room temper ature | room temper ature | room tempe rature | roo m temp eratu re | room temp eratu re | room temper ature | roo m tem pera ture |
| | mixing temperatu re | 110 | 105 | 70 | 140 | 90 | 75 | 70 | 140 | 90 | 75 |
| | degassing | 110 | 105 | 70 | 140 | 90 | 75 | 70 | 140 | 90 | 75 |
| | extrusion temperatu re | 90 | 85 | 57 | 120 | 70 | 55 | 57 | 120 | 70 | 55 |
| | die orifice temperatu re | 52 | 65 | 67 | 69 | 67 | 71 | 67 | 69 | 67 | 71 |

### Preparation Method:

Weighing: The API and PLGA indicated in the table above were accurately weighed, with the feed amount of API calculated based on the free compound of pramipexole. The molar ratio of pramipexole to pamoic acid was 2:1, and the molar ratio of pramipexole to the various anions of the remaining salts in Table 2 was 1:1. After a physical mixture of the two was achieved, it was added to the feed hopper of a hot melt extruder.

Extrusion: A Thermo Fisher Pharma 11 with parallel counter-rotating twin screw was employed, and the hot melt extruder was initiated. The feed temperature, pushing temperature, mixing, degassing, extrusion, and die orifice temperature were set according to the above table, with a pressure threshold set at 60 bar, extrusion speed at 100 rpm, and torque threshold at 8 N.cm. Once these settings were set, automatic feeding, mixing, and extrusion began.

Granulation: The die orifice specification was set at 3mm, and the prilling length was set at 35mm for extrusion. After extrusion (the first drop of extrudate), a glass rod was manually used to draw the strands to the pelletizer's roller, and granulation cutting was initiated.

Milling: The granulated particles were transferred into a ball mill to begin grinding. Samples were taken every 5 minutes to monitor the particle size. When the target particle size (D₅₀ = 50~100 µm) was reached, grinding was ceased, and sustained-release particles were obtained.

### Example 3: Preparation of a Subcutaneous Implant Containing Pramipexole

**Table 3: Subcutaneous Implant Formulation Feed Table**

| Formulation No. | | 35 | 36 | 37 | 38 | 59 | 60 | 61 | 62 |
|---|---|---|---|---|---|---|---|---|---|
| Salt Type | | erucate | pamoat e | stearate | oleate | erucate | pamoat e | palmitat e | oleate |
| Start ing Mate rial | API (g) | 5.5 | 3.6 | 4.7 | 4.7 | 13 | 7.5 | 10.5 | 10 |
| | Melting Point | 58 | 142 | 82 | 32 | 58 | 142 | 82 | 32 |
| Exci pient | PLGA Type | 8515 4.5A | 7525 5E | 7525 5A | 7525 5E | 8515 2.5A | 7525 7E | 75257A | 7525 7E |
| | Glass Transition Temperatu re | 47 | 49 | 47 | 51 | 47 | 49 | 47 | 51 |
| | PLGA (g) | 12 | 12 | 12 | 12 | 12 | 12 | 12 | 12 |
| Theoretical Drug Loading | | 31.4% | 23.1% | 28.1% | 28.1% | 52% | 38.5% | 46.7% | 45.5% |
| Tern perat ure Zone Setti ng (°C) | feed temperatur e | room temperat ure | room temper ature | room temperat ure | room temperat ure | room temperat ure | room temper ature | room temperat ure | room tempera ture |
| | pushing temperatur e | room temperat ure | room temper ature | room temperat ure | room temperat ure | room temperat ure | room temper ature | room temperat ure | room tempera ture |
| | mixing temperatur e | 70 | 140 | 90 | 75 | 70 | 140 | 90 | 75 |
| | degassing | 70 | 140 | 90 | 75 | 70 | 140 | 90 | 75 |
| | extrusion temperatur e | 57 | 120 | 70 | 55 | 57 | 120 | 70 | 55 |
| | die orifice temperatur e | 67 | 69 | 67 | 71 | 67 | 69 | 67 | 71 |
| Die Orifi ce Spec ificat ion | die orifice size | 3mm | 3mm | 3mm | 3mm | 3mm | 3mm | 3mm | 3mm |
| | prilling length | 35mm | 35mm | 35mm | 35mm | 35mm | 35mm | 35mm | 35mm |

### Preparation Method:

Weighing: The API and PLGA indicated in the table above were accurately weighed, with the feed amount of API based on the free compound of pramipexole. The molar ratio of pramipexole to pamoic acid was 2:1, and the molar ratio of pramipexole to the various anions of the remaining salts in Table 3 was 1:1. Following their physical mixture, the combined materials were added to the feed hopper of a hot melt extruder.

Extrusion: The Thermo Fisher Pharma 11 with parallel counter-rotating twin screws was employed, and the hot melt extruder was initiated. The feed temperature, pushing temperature, mixing, degassing, extrusion, and die orifice temperature were set according to the above table, with a pressure threshold of 60 bar, an extrusion speed of 100 rpm, and a torque threshold of 8 N.cm. Once these parameters were set, automatic feeding, mixing, and extrusion began.

Granulation: The die orifice specifications listed in the table above were employed, and the prilling length was set according to the table above. After extrusion (the first droplet of extrudate), a glass rod was manually used to draw the strands to the pelletizer's roller. The cutting and granulating process was initiated, resulting in the formation of subcutaneous implants.

### Comparison Example 1: Preparation of Microsphere Formulation Containing Pramipexole

**Table 4: Comparative Microsphere Formulation Ingredient Table**

| Formulation No. | | Comparative 1 | Comparative 2 | Comparati ve 3 | Comparative 4 | Comparative 5 |
|---|---|---|---|---|---|---|
| Salt Type | | hydrochlorid e | free compound | behenate | pamoate | pamoate |
| Oil Phase 1 | API (g) | 2.5 | 2.5 | 2.5 | 1 | 12 |
| | Type of Solvent 1 | methanol | methanol | / | DMSO | DMSO |
| | Solvent 1 (ml) | 15 | 15 | 15 | 15 | 15 |
| Oil phase 2 | PLGA Type | 5050 4.5A | 5050 4.5A | 5050 4.5A | 5050 4.5A | 5050 4.5A |
| | PLGA (g) | 12 | 12 | 12 | 12 | 12 |
| | Type of Solvent 2 | dichloromet hane | dichloromet hane | dichlorom ethane | dichloromet hane | dichlorometh ane |
| | Solvent 2 (ml) | 35 | 35 | 35 | 35 | 35 |
| External Aqueous Phase | PVA (g) | 30 | 30 | 30 | 30 | 30 |
| | Water (L) | 3 | 3 | 3 | 3 | 3 |
| Theoretical drug loading | | 17.2% | 17.2% | 17.2% | 7.7% | 50.0% |

The comparative examples 1 to 5 in the table above were prepared according to the method of Example 1, with the molar ratio of pramipexole to pamoic acid being 2:1, and the molar ratio of pramipexole to hydrochloric acid or behenic acid being 1:1. During the preparation of comparative example 3, a variety of solvents including dichloromethane, chloroform, ethyl acetate, methanol, ethanol, isopropanol, *tert*-butanol, DMSO, DMF, acetone, and tetrahydrofuran were used as Solvent 1 to dissolve the behenate salt of pramipexole, but none were able to effectively dissolve it, thus failing to prepare the microsphere formulation.

### Experimental Example 1: Quality Examination of Microsphere Formulations

Examination Method: the actual drug loading, encapsulation efficiency, particle size, residual solvent content, and *in vitro* release profiles of the microsphere examples and comparative samples mentioned above were examined.

Method for *In vitro* Release Assay: The microspheres were placed in a vial, a pH 7.4±0.1 PBS buffer solution was added, and the solution was placed in a 37±0.5°C water bath shaker. Samples were taken at different time points, and HPLC was employed to measure the drug content released into the buffer solution.

In this test, *"in vitro* burst release" refers to the cumulative release on the first day of *in vitro* release. The test results are as follows:

**Table 5: Quality Examination Results of Microsphere Formulation Examples**

| Formulation No. | | 1 | 2 | 19 | 21 | 22 | 45 |
|---|---|---|---|---|---|---|---|
| Salt Type | | heptadecanoate | palmitate | erucate | stearate | oleate | palmitate |
| Actual Drug Loading | | 10.49% | 24.175% | 30.57% | 24.87% | 17.75% | 39.44% |
| Encapsulation Efficiency | | 94.5% | 96.7% | 86.6% | 88.5% | 88.8% | 84.5% |
| Particle Size | D₅₀ | 81 | 65 | 81.04 | 82.88 | 52.77 | 81.82 |
| | Span | 1.68 | 1.47 | 1.52 | 1.75 | 1.39 | 1.68 |
| *in vitro* burst release | | 0.74% | 0.65% | 0.52% | 0.54% | 0.99% | 0.50% |
| Residual Solvent | Water | 0.49% | 0.41% | 0.55% | 0.45% | 0.36% | 0.50% |
| | Solvent 2 | 133ppm | 35ppm | 53ppm | 103ppm | 33ppm | 63ppm |
| | Solvent 1 | 133ppm | 0.51% | 0.61% | 0.73% | 0.25% | 0.40% |

**Table 6: Quality Examination Results of Microsphere Formulation Comparative Samples**

| Formulation No. | | Comparati ve 1 | Comparative 2 | Comparative 4 | Comparative 5 |
|---|---|---|---|---|---|
| Appearance | | porous surface | crystals visible on the surface | spherical, well-rounded, and dense | porous surface, loose structure |
| Salt Type | | hydrochlo ride | free compound | pamoate | pamoate |
| Actual Drug Loading | | 7.23% | 4.95% | 6.57% | 32.51% |
| Encapsulation Efficiency | | 42.01% | 28.77% | 85.32% | 65.02% |
| | D₅₀ | 79 | 81 | 63 | 65 |
| Particle Size | Span | 1.59 | 1.67 | 1.61 | 1.60 |
| *in vitro* burst release | | 58% | 73% | 12.16% | 18.89% |
| Residual Solvent | Water | 0.52% | 0.47% | 0.49% | 0.38% |
| | Solve nt 2 | 128ppm | 139ppm | 68ppm | 61ppm |
| | Solve nt 1 | 128ppm | 41ppm | 0.47% | 0.41% |

Examination findings: The microspheres prepared in Examples 1, 2, 19, 21, 22, and 45 displayed high encapsulation efficiencies and virtually no burst release. In contrast, Comparative Examples 1 and 2 (hydrochloride salt, free compound) during preparation showed a significant migration of API leading to seriously low encapsulation efficiencies, and this was accompanied by a substantial burst release and poor appearance. Comparative Example 5 microspheres had poor sphericity, were porous, loosely structured, and the *in vitro* release curve was unstable; Comparative Example 4 exhibited a large burst release, posing a clinical risk.

### Experimental Example 2: Quality Examination of Sustained-Release Particle Formulations

Examination Method: the actual drug loading, encapsulation efficiency, particle size, and *in vitro* release of the sustained-release particle example samples mentioned above were examined. The test results are as follows:

**Table 7: Quality Examination Results of Sustained-Release Particles**

| Formulation No. | | 10 | 11 | 27 | 28 | 29 | 30 | 52 | 53 |
|---|---|---|---|---|---|---|---|---|---|
| Salt Type | | heptadecan oate | palmit ate | eruca te | pamo ate | steara te | oleat e | pamo ate | palmit ate |
| Actual Drug Loading | | 23.57% | 24.53 % | 30.10 % | 22.00 % | 26.90 % | 27.60 % | 36.81 % | 44.69 % |
| Encapsulation Efficiency | | 98.2% | 98.1% | 95.9 % | 95.2 % | 95.7 % | 98.2 % | 95.6 % | 95.7% |
| Particle Size | D₅₀ | 72 | 79 | 56.09 | 59.03 | 60.14 | 57.83 | 59.82 | 60.9 |
| | Spa n | 1.55 | 1.39 | 1.51 | 1.39 | 1.42 | 1.47 | 1.66 | 1.46 |
| *in vitro* burst release | | 1.48% | 1.30% | 2.85 % | 3.79 % | 1.05 % | 1.43 % | 1.07 % | 1.93% |
| Molecular Weight of PLGA (Da) | M w | 29832 | 55250 | 5950 0 | 60500 | 4250 0 | 4310 0 | 97750 | 10112 0 |
| | PD I | 1.60 | 1.73 | 1.82 | 1.78 | 1.89 | 1.71 | 1.65 | 1.84 |

According to the data in the above table, it can be seen that the sustained-release particle samples prepared in each example meet the required pharmaceutical specifications, with the amount of burst release being at a low level.

### Experimental Example 3: Quality Examination of Subcutaneous Implants

Examination Method: the actual drug loading, encapsulation efficiency and *in vitro* release of the subcutaneous implant example samples mentioned above were examined. The test results are as follows:

**Table 8: Quality Examination Results of Subcutaneous Implants**

| Formulation No. | | 35 | 36 | 37 | 38 | 60 | 61 |
|---|---|---|---|---|---|---|---|
| Salt Type | | erucate | pamoate | stearate | oleate | pamoate | palmitate |
| Actual Drug Loading | | 29.39% | 21.69% | 26.75% | 27.06% | 26.46% | 28.53% |
| Encapsulation Efficiency | | 93.6% | 93.9% | 95.2% | 96.3% | 68.7% | 61.1% |
| *in vitro* burst release | | 1.94% | 2.58% | 0.71% | 0.97% | 0.71% | 0.52% |
| Molecular Weight of PLGA (Da) | Mw | 60690 | 61710 | 43350 | 43962 | 99705 | 101120 |
| | PDI | 1.77 | 1.73 | 1.83 | 1.66 | 1.58 | 1.84 |

According to the data in the above table, it can be seen that the subcutaneous implants prepared in each example meet the required pharmaceutical specifications, with the burst release being at a low level.

### Experimental Example 4: In Vivo Pharmacokinetic Study

Male rats weighing approximately 250g were selected and randomly divided into groups, with 5 in each group. The drugs were administered to them via intramuscular injection, only once. Blood was taken from the veins before and after the administration of the drugs to determine the concentration of pramipexole in the plasma after administration, and to plot the drug-time curve for the calculation of pharmacokinetic parameters.

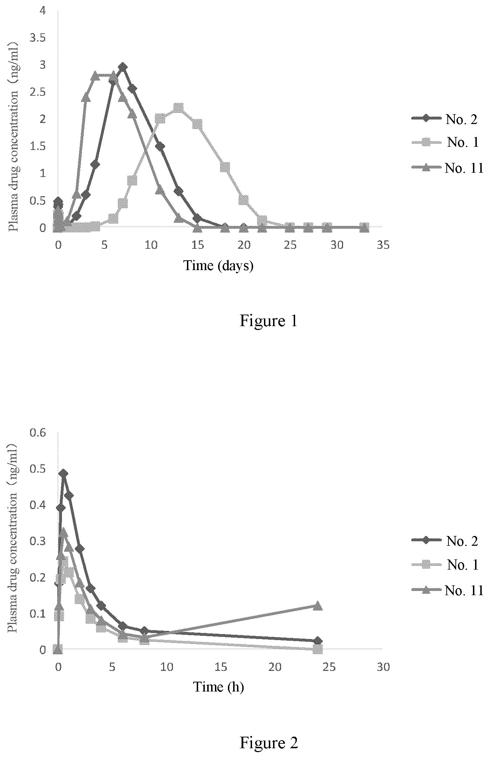

Protocol 1: The test samples were examples samples numbered as formulation No. 1, 2, and 11; the dose of administration was 4mg/kg, and the test results are shown in Figure 1 and Figure 2.

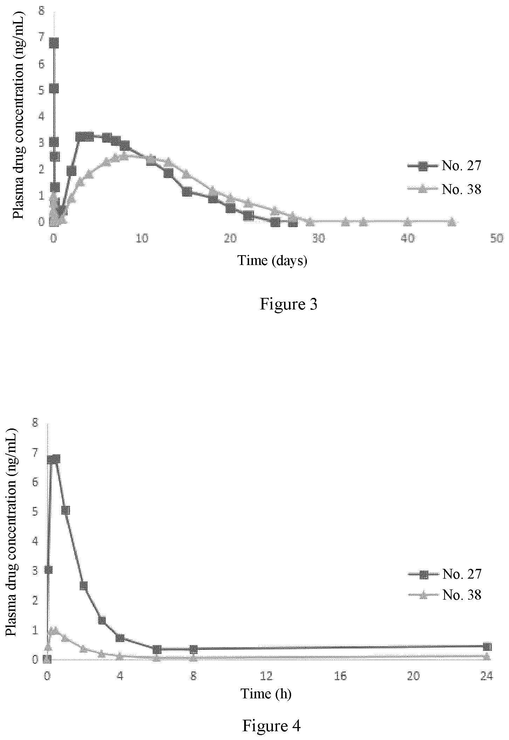

Protocol 2: The test samples were examples samples numbered as formulation No. 27 and 38; the dose of administration was 8mg/kg, and the test results are shown in Figure 3 and Figure 4.

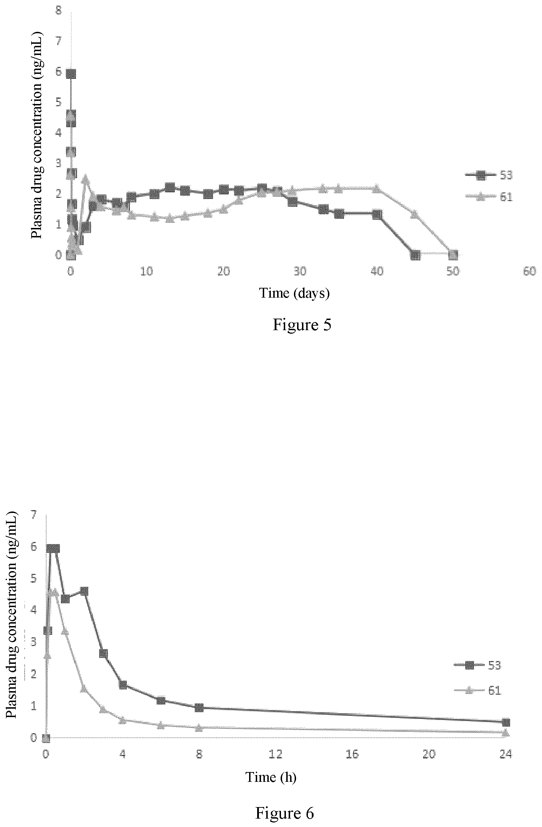

Protocol 3: The test samples were examples samples numbered as formulation No. 53 and 61; the dose of administration was 16mg/kg, and the test results are shown in Figure 5 and Figure 6.

**Table 9: In Vivo PK Test Data of Example Samples in Rat**

| Formulation No. | 1 | 2 | 11 | 27 | 38 | 53 | 61 |
|---|---|---|---|---|---|---|---|
| API | heptadeca noate | palmitate | palmitate | erucate | oleate | palmitate | palmitate |
| Dosage Form | microsph ere | microsph ere | sustained -release particle | sustained-release particle | subcutane ous implant | sustained -release particle | subcutane ous implant |
| AUC ₀₋₂₄ (day*ng/ml) | 0.038 | 0.084 | 0.091 | 0.85 | 0.13 | 1.32 | 0.57 |
| AUC ₀₋ₗₐₛₜ (day*ng/ml) | 20.68 | 20.30 | 20.29 | 41.10 | 39.39 | 74.51 | 79.98 |
| AUC_{0-∞} | 22.12 | 21.71 | 21.90 | 42.21 | 42.11 | 79.66 | 83.51 |
| AUC₀₋ₗₐₛₜ/AUC_{0-∞} | 93.49% | 93.51% | 92.65% | 97.37% | 93.54% | 93.54% | 95.77% |
| *In vivo* burst release | 0.18% | 0.41% | 0.45% | 2.07% | 0.34% | 1.77% | 0.71% |

| | | | | | | | |
|---|---|---|---|---|---|---|---|
| Note: *"In vivo* burst release" refers to the proportion of the exposure on the first day to the total exposure over the entire period. The calculation formula for *in vivo* burst release is: **In vivo** burst release = AUC₀₋₂₄ ÷ AUC₀₋ₗₐₛₜ * 100%. | | | | | | | |

According to the above test results, it can be seen that the long-acting sustained-release formulation of pramipexole prepared by the present invention is capable of achieving stable release over an extended period of 1 week, 2 weeks, or even 1 month without significant burst release. The plasma drug concentration remains stable, fulfilling the requirements for clinical efficacy and medication safety, and is able to significantly improve patient compliance.

The present invention provides a long-acting sustained-release formulation containing a pharmaceutically acceptable salt of pramipexole and preparation method therefor. A person skilled in the art can implement these by referencing the content of this document and appropriately modifying the starting materials, process parameters, and other factors. The methods and products of the present invention have been described through preferred examples, and it is evident to those skilled in the relevant technical fields that, without departing from the content, spirit and scope of the present invention, modifications, appropriate changes, and combinations to the methods and products described herein can be made to realize the technology of the present invention. It is particularly noted that all such similar substitutions and modifications are apparent to those skilled in the art and are considered to be within the spirit, scope, and content of the present invention.

## Claims

1. A long-acting sustained-release formulation containing pramipexole, **characterized in that** it comprises a pharmaceutically acceptable salt of pramipexole and a pharmaceutical high polymer material;
the formulation is selected from a microsphere, a sustained-release particle, and a subcutaneous implant;
the pharmaceutically acceptable salt of pramipexole is selected from erucate, stearate, oleate, α-linolenate, heptadecanoate, pamoate, and palmitate; preferably, the pharmaceutically acceptable salt of pramipexole is selected from erucate, stearate, oleate, α-linolenate, heptadecanoate, and palmitate; more preferably, the pharmaceutically acceptable salt of pramipexole is selected from erucate, stearate, oleate, heptadecanoate, and palmitate;
the high polymer material is selected from PLGA, PLA, mPEG-PLA, and PEG-PLA-PEG.

2. The long-acting sustained-release formulation according to claim 1, **characterized in that** the high polymer material is PLGA, with a molecular weight of 10000~100000 Da, wherein the LA:GA block ratio of PLGA is 5:95~95:5; preferably, the high polymer material is PLGA, with a molecular weight of 25000~100000 Da, wherein the LA:GA block ratio of PLGA is 50:50~85:15;
or, the high polymer material is PLA, with a molecular weight of 10000~100000 Da;
or, the high polymer material is mPEG-PLA, wherein the molecular weight of mPEG in mPEG-PLA is 1000~4000 Da, and the molecular weight of PLA is 10000~100000 Da;
or, the high polymer material is PEG-PLA-PEG, wherein the molecular weight of PEG is 1000~4000 Da, and the molecular weight of PLA is 10000~100000 Da.

3. The long-acting sustained-release formulation according to claim 1 or 2, **characterized in that** the formulation is in the form of microspheres, with the active ingredient being pramipexole heptadecanoate or palmitate; each unit formulation comprises 1~3 parts of pramipexole calculated as the free compound and 9~7 parts of PLGA, with the LA:GA block ratio of PLGA being 50:50, and the theoretical drug loading is 10%~30%, preferably, the theoretical drug loading is 15%~25%.

4. The long-acting sustained-release formulation according to claim 1 or 2, **characterized in that** the formulation is in the form of microspheres, with the active ingredient being pramipexole erucate, stearate or oleate; each unit formulation comprises 2~4 parts of pramipexole calculated as the free compound and 8~6 parts of PLGA, with the LA:GA block ratio of PLGA being 75:25-85:15, and the theoretical drug loading is 20%~40%, preferably, the theoretical drug loading is 25%~35%.

5. The long-acting sustained-release formulation according to claim 1 or 2, **characterized in that** the formulation is in the form of microspheres, with the active ingredient being pramipexole erucate, palmitate or oleate; each unit formulation comprises 3~5.5 parts of pramipexole calculated as the free compound and 7~4.5 parts of PLGA, with the LA:GA block ratio of PLGA being 75:25-85:15, and the theoretical drug loading is 30%~55%, preferably, the theoretical drug loading is 35%~50%, and more preferably, the theoretical drug loading is 35%~45%.

6. The long-acting sustained-release formulation according to claim 1 or 2, **characterized in that** the formulation is in the form of sustained-release particles, with the active ingredient being pramipexole erucate, pamoate, stearate, oleate, palmitate, or heptadecanoate; each unit formulation comprises 1~5.5 parts of pramipexole calculated as the free compound and 9~4.5 parts of PLGA, with the LA:GA block ratio of PLGA being 50:50-85:15, and the theoretical drug loading is 20%~55%.

7. The long-acting sustained-release formulation according to claim 1 or 2, **characterized in that** the formulation is in the form of a subcutaneous implant, with the active ingredient being pramipexole erucate, pamoate, stearate, oleate, or palmitate; each unit formulation comprises 2~5.5 parts of pramipexole calculated as the free compound and 8~4.5 parts of PLGA, with the LA:GA block ratio of PLGA being 50:50-85:15, and the theoretical drug loading is 20%~55%.

8. A method for preparing the microspheres according to any one of claims 3-5, comprising the following steps:
oil phase preparation: dissolving a pharmaceutically acceptable salt of pramipexole in a first solvent to obtain a first oil phase; dissolving PLGA in a second solvent to obtain a second oil phase;
external aqueous phase preparation: dissolving PVA in water for later use;
oil phase mixing: stirring and mixing the first oil phase and the second oil phase to obtain a mixed oil phase;
emulsification: filling the external aqueous phase into an inline shear machine and mixing it with the mixed oil phase at a speed of 5000 to 13000 rpm for shear emulsification;
solidification: evaporating the solvent from the sheared solution under stirring and heating according to a temperature increase curve to solidify and then lyophilizing to form the microspheres;
preferably, during the solidification step, evaporating the solvent from the sheared solution under stirring and heating according to a temperature increase curve to solidify, sieving, and then lyophilizing to form the microspheres.

9. A method for preparing the sustained-release particles according to claim 6, comprising the following steps:
mixing a pharmaceutically acceptable salt of pramipexole with PLGA and adding the mixture to the feed hopper of a hot melt extruder;
extrusion: using a hot melt extruder to extrude at an extrusion speed of 100 rpm under a pressure of 60 bar, controlling the torque at 7~8 N.cm;
granulating: traction drawing after extrusion, and granulating with a pelletizer;
milling: crushing the granulated product with a low-temperature ball mill to a size of 50~100µm, preferably 60~100µm, to obtain sustained-release particles.

10. A method for preparing the subcutaneous implant according to claim 7, comprising the following steps:
mixing a pharmaceutically acceptable salt of pramipexole with PLGA and adding the mixture to the feed hopper of a hot melt extruder;
extrusion: using a hot melt extruder to extrude at an extrusion speed of 100 rpm under a pressure of 60 bar, controlling the torque at 7~8 N.cm;
granulating: traction drawing after extrusion, and granulating with a pelletizer to obtain the subcutaneous implant.
